(19) Europäisches Patentamt
European Patent Office
Office européen des brevets

(11) EP 1 218 382 B1

(12) EUROPÄISCHE PATENTSCHRIFT

(45) Veröffentlichungstag und Bekanntmachung des Hinweises auf die Patenterteilung:
**28.01.2004 Patentblatt 2004/05**

(21) Anmeldenummer: **00967692.5**

(22) Anmeldetag: **18.09.2000**

(51) Int Cl.[7]: **C07D 487/04**, A61K 31/435, A61K 31/495, A61K 31/505, A61K 31/53, A61P 25/00, A61P 43/00

(86) Internationale Anmeldenummer:
**PCT/EP2000/009096**

(87) Internationale Veröffentlichungsnummer:
**WO 2001/027111 (19.04.2001 Gazette 2001/16)**

(54) **BICYCLISCHE IMIDAZO-3-YL-AMINDERIVATE**
BICYCLIC IMIDAZO-3-YL-AMINE DERIVATIVES
DERIVES D'IMIDAZO-3-YL-AMINE BICYCLIQUES

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **08.10.1999 DE 19948434**
**08.10.1999 DE 19948438**

(43) Veröffentlichungstag der Anmeldung:
**03.07.2002 Patentblatt 2002/27**

(73) Patentinhaber: **Grünenthal GmbH**
**52078 Aachen (DE)**

(72) Erfinder:
- **GERLACH, Matthias**
  **63636 Brachttal (DE)**
- **MAUL, Corinna**
  **52066 Aachen (DE)**

(56) Entgegenhaltungen:
**EP-A- 0 266 890** **EP-A- 0 518 033**

- **RIMOLI MG AVALLONE L DE CAPRARIIS P LURASCHI E ABIGNENTE E FILIPPELLI W BERRINO L ROSSI F: "Research on heterocyclic compounds. XXXVII. Synthesis and antiinflammatory activity of methyl-substituted imidazo[1,2-a]pyrazine derivatives" EUROPEAN JOURNAL OF MEDICINAL CHEMISTRY.CHIMICA THERAPEUTICA,FR,EDITIONS SCIENTIFIQUE ELSEVIER, PARIS, Bd. 32, Nr. 3, 1997, Seiten 195-203, XP004075421 ISSN: 0223-5234**
- **BLACKBURN C: "A Three-Component Solid-Phase Synthesis of 3-Aminoimidazo[1,2-a]azines" TETRAHEDRON LETTERS,NL,ELSEVIER SCIENCE PUBLISHERS, AMSTERDAM, Bd. 39, Nr. 31, 30. Juli 1998 (1998-07-30), Seiten 5469-5472, XP004124091 ISSN: 0040-4039 in der Anmeldung erwähnt**
- **BLACKBURN C ET AL: "Parallel Synthesis of 3-Aminoimidazo[1,2-a]pyridines and pyrazines by a New Three-Component Condensation" TETRAHEDRON LETTERS,NL,ELSEVIER SCIENCE PUBLISHERS, AMSTERDAM, Bd. 39, Nr. 22, 28. Mai 1998 (1998-05-28), Seiten 3635-3638, XP004118699 ISSN: 0040-4039 in der Anmeldung erwähnt**
- **H.BIENAYME ET AL.: "A New Heterocyclic Multicomponent Reaction For the Combinatorial Synthesis of Fused 3-Aminoimidazoles." ANGEW. CHEM. INTL. ED., Bd. 37, Nr. 16, 1998, Seiten 2234-2237, XP000978871 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft substituierte bicyclische Imidazo-3-yl-amine und Arzneimittel enthaltend diese Verbindungen.

Für einzelne Verbindungen aus der Klasse der Imidazo-3-yl-amine sind interessante pharmakologische Eigenschaften bekannt. So werden bestimmte Imidazo[1,2-a]pyridine als den Blutdruck senkende Wirkstoffe (GB-B-1,135,893), als Anthelminthika und Antimykotika (J. Med. Chem. 1972, 15, 982-985)und als antisekretorische Wirkstoffe zur Behandlung von entzündlichen Erkrankungen (EP-A-0 068 378) beschrieben. Eine Wirkung einzelner Imidazopyridine gegen entzündliche Erkrankungen insbesondere des Magens beschreiben auch EP-A-0 266 890 und J. Med. Chem. 1987, 30, 2031-2046. Weitere, für einzelne Vertreter aus der Klasse der Imidazo-3-yl-amine beschriebene pharmakologische Wirkungen sind antibakterielle Eigenschaften (Chem. Pharm. Bull. 1992, 40, 1170), antivirale Eigenschaften (J. Med. Chem. 1998, 41, 5108-5112) sowie die Wirkung als Benzodiazepin-Rezeptor Antagonist (J. Heterocyclic Chem. 1998,35, 1205-1217).

Angesichts dieser interessanten Wirkungen wurden in der Vergangenheit verschiedene Vertreter aus der Klasse der substituierten Imidazo-3-yl-amine synthetisiert. Insbesondere wurde versucht, die Zahl der verfügbaren substiuierten Imidazo-3-yl-amine durch kombinatorische Syntheseverfahren zu vergrößern. So beschreiben C.

Blackburn et al. in Tetrahedron Lett. 1998, 39, 5469-5472 eine Dreikomponenten-Festphasensynthese zur Herstellung von Imidazo-3-yl-aminen und in Tetrahedron Lett. 1998, 39, 3635-3638 eine Dreikomponenten-Kondensation zur Parallelsynthese von Imidazo-3-yl-aminen. Ähnlich der letztgenannten Reaktion ist die in von K. Groebke et al.in Synlett 1998, 661-663 publizierte Synthese. Eine Mehrkomponentenreaktion für die kombinatorische Synthese von Imidazo-3-yl-aminen, mit der auch vereinzelte Imidazo-5-amine herstellt wurden, beschreiben auch H. Bienayme und K. Bouzid in Angew. Chem. 1998, 110 (16), 2349-2352.

Die gemäß dem Stand der Technik mögliche Variationsbreite der Substituenten am Amino-Stickstoff und in der 2-Position des Imidazolrings war jedoch begrenzt.

Der vorliegenden Erfindung lag daher die Aufgabe zugrunde, weitere bicyclischen Imidazo-3-yl-amine, und diese enthaltende Arzneimittel bereitzustellen.

Gegenstand der Erfindung sind daher bicyclische Imidazo-3-yl-amine der allgemeinen Formel I,

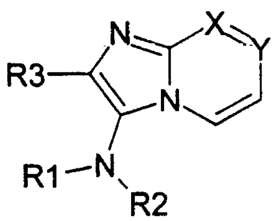

I

worin

X und Y CH oder N bedeuten mit der Maßgabe, daß X und Y nicht gleichzeitig N bedeuten,

$R^1$ *tert*-Butyl, $(CH_2)_nCN$ mit n = 4 ,5 oder 6, gegebenenfalls substituiertes Phenyl, $C_4$-$C_8$-Cycloalkyl, $CH_2CH_2R$ (R = 4-Morpholino), 1, 1, 3, 3-Tetramethylbutyl oder $CH_2R^a$, wobei $R^a$ für Wasserstoff, OH, $C_1$-$C_8$-Alkyl (verzweigt oder unverzweigt), gegebenenfalls substituiertes Phenyl, CO(OR') (mit R' = unverzweigtes $C_1$-$C_4$-Alkyl oder verzweigtes $C_1$-$C_5$-Alkyl), $PO(OR')_2$ (mit R' = unverzweigtes $C_1$-$C_4$-Alkyl oder verzweigtes $C_1$-$C_5$-Alkyl) oder $Si(R^xR^yR^z)$ (mit $R^x$, $R^y$, und $R^z$ jeweils unabhängig voneinander $C_1$-$C_4$-Alkyl(verzweigt oder unverzweigt), $C_4$-$C_8$-Cycloalkyl oder Phenyl) steht, bedeutet,

$R^2$ Wasserstoff, $COR^b$, wobei $R^b$ für $C_1$-$C_4$-Alkyl (verzweigt oder unverzweigt)oder $C_3$-$C_8$-Cycloalkyl steht, $CH_2CH_2CO(OR^c)$, wobei $R^c$ für $C_1$-$C_4$-Alkyl (verzweigt oder unverzweigt), Adamantyl, gegebenenfalls substituiertes Phenyl, gegebenenfalls substituiertes 1-Naphtyl oder 2-Naphtyl oder jeweils gegebenenfalls substiuiertes 2-Pyridyl, 3-Pyridyl, 4-Pyridyl, Thiazolyl oder Furoyl steht, $CH_2$Phenyl, $CH_2CH_2R^d$, wobei $R^d$ für gegebenenfalls substituiertes Phenyl steht, oder $CONHR^e$, wobei $R^e$ für $C_1$-$C_8$-Alkyl (verzweigt oder unverzweigt), $C_3$-$C_8$-Cycloalkyl oder gegebenenfalls substituiertes Phenyl steht, bedeutet,

$R^3$ Methyl, Ethyl, *tert*-Butyl, $C_3$-$C_8$-Cycloalkyl, Phenyl, gegebenenfalls einfach substituiert in 3-, 5- oder 6-Position oder gegebenenfalls mehrfach substituiert in 4-Position und zusätzlich in 2- und/oder 3- und/oder 5-und/oder 6-Position, Phenoxy, gegebenenfalls substituiertes Naphtyl, gegebenenfalls substituiertes Pyrrol, gegebenenfalls substituiertes Pyridyl, gegebenenfalls substituiertes Furan, gegebenenfalls substituiertes Thiophen, gegebenenfalls substituiertes Anthracen, gegebenenfalls substituiertes Phenanthren oder gegebenenfalls substituiertes Chinolin bedeutet,

mit der Maßgabe, daß $R^3$ nicht n-Propyl, Cyclohexyl, unsubstituiertes Phenyl oder in 3-Position mit einer Carbonsäureamid-Gruppe monosubstituiertes Phenyl bedeutet, wenn $R^1$ t-Butyl, n-Propyl, n-Butyl, 1,1,3,3-Tetramethylbutyl, Cyclohexyl, $CH_2CH_2R$ (R = 4-Morpholino), monosubstituiertes Phenyl, 2,6,-Dimethylphenyl oder Benzyl bedeutet und gleichzeitig $R^2$ Wasserstoff oder -CO(Methyl) bedeutet, und daß $R^2$ nicht Wasserstoff bedeutet, wenn gleichzeitig $R^1$ Benzyl und $R^3$ Methyl bedeutet oder gleichzeitig $R^1$ $CH_2C(O)$*tert*-Butyl und $R^3$ unsubstituiertes Phenyl bedeutet, in Form der Basen oder von pharmazeutisch akzeptablen Salzen.

Erfindungsgemäß bevorzugt sind dabei solche Verbindungen, bei denen $R^2$ Wasserstoff bedeutet, $R^1$ ausgewählt ist aus der Gruppe $(CH_2)_nCN$ mit n = 4 ,5 oder 6, Cyclohexyl, $CH_2CO(OMethyl)$, 2,6-Dimethylphenyl, 1,1,3,3,-Tetramethylbutyl, *tert*-Butyl oder n-Butyl und $R^3$ ausgewählt ist aus der Gruppe 2-Pyridyl, 3-Pyridyl, 2-Furanyl, 2-Pyrroyl, Methyl, *tert*-Butyl, 3-Hydroxyphenyl, 3,4-Dimethoxyphenyl, 2,3-Dichlorphenyl, 2,4-Dichlorphenyl, 2-Methoxyphenyl, 2,3-Dimethoxyphenyl, 3-Bromphenyl, 4-Brom-2-fluorphenyl, 5-Brom-2-fluorphenyl, 3-Brom-4-fluorphenyl, 3-Chlorphenyl, 3,4-Dichlorphenyl, 3-Fluorphenyl, 3-Methylphenyl, 3-Phenoxyphenyl, 3-(4-Chlorphenoxy)phenyl, 2-Chlor-4-fluorphenyl, 2-Chlor-6-fluorphenyl, 2,4-Dimethylphenyl, 2,5-Dimethylphenyl, 2-Bromphenyl, 2-Fluorphenyl oder 2-(Trifluormethyl)-phenyl.

Besonders bevorzugt sind erfindungsgemäß bicyclische Imidazo-3-yl-amine ausgewählt aus der Gruppe

(6-Isocyano-hexyl)-(2-pyridin-2-yl-imidazo[1,2-a]pyridin-3-yl)-amin,
(2-Furan-2-yl-imidazo[1,2-a]pyridin-3-yl)-(6-isocyanohexyl)-amin,
(2-Cyclohexyl-imidazo[1,2-a]pyrazin-3-yl)-(6-isocyanohexyl)-amin,
(2,6-Dimethyl-phenyl)-(2-furan-2-yl-imidazo[1,2-a]pyridin-3-yl)-amin,
(2-Furan-2-yl-imidazo[1,2-a]pyrazin-3-ylamino)-essigsäuremethylester,
(2-Cyclohexyl-imidazo[1,2-a]pyrimidin-3-ylamino)-essigsäuremethylester,
(2-Methyl-imidazo[1,2-a]pyrazin-3-ylamino)-essigsäuremethylester,
(2-Pyridin-4-yl-imidazo[1,2-a]pyrazin-3-yl)-(1,1,3,3-tetramethyl-butyl)-amin,
(2-Methyl-imidazo[1,2-a]pyrazin-3-yl)-(1,1,3,3-tetramethyl-butyl)-amin,
3-(3-*tert*-Butylamino-imidazo[1,2-a]pyridin-2-yl)-phenol,
Butyl-[2-(2,3-dichlor-phenyl)-imidazo[1,2-a]pyrazin-3-yl]-amin,
[(2-Phenyl-imidazo[1,2-a]pyridin-3-ylamino)-methyl)-phosphonsäurediethylester,
*tert*-Butyl-(2-*tert*-butyl-imidazo[1,2-a]pyridin-3-yl)-amin,
Butyl-(2-o-tolyl-imidazo[1,2-a]pyrimidin-3-yl)-amin, (2,6-Dimethyl-phenyl)-[2-(2-methoxy-phenyl)-imidazo[1,2-a]pyrazin-3-yl]-amin,
Butyl-(2-o-tolyl-imidazo[1,2-a]pyrimidin-3-yl)-amin, *tert*-Butyl-(2-pyridin-3-yl-imidazo[1,2-a]pyrimidin-3-yl)-amin,
*tert*-Butyl-(2-methyl-imidazo[1,2-a]pyridin-3-yl)-amin, [2-(1H-Pyrrol-2-yl)-imidazo[1,2-a]pyrimidin-3-yl]-(1,1,3,3-tetramethyl-butyl)-amin,
Cyclohexyl-(2-furan-2-yl-imidazo[1,2-a]pyridin-3-yl)-amin,
*tert*-Butyl-(2-pyridin-3-yl-imidazo[1,2-a]pyridin-3-yl)-amin,
*tert*-Butyl-(2-pyridin-2-yl-imidazo[1,2-a]pyridin-3-yl)-amin,
*tert*-Butyl-(2-thiophen-2-yl-imidazo[1,2-a]pyridin-3-yl)-amin,
Cyclohexyl-(2-methyl-imidazo[1,2-a]pyridin-3-yl)-amin, N-Cyclohexyl-N-[2-(5-methyl-furan-2-yl)-imidazo[1,2-a]pyridin-3-yl]-acetamid,
*tert*-Butyl-[2-(5-methylsulfanyl-thiophen-2-yl)-imidazo[1,2-a]pyrimidin-3-yl]-amin,
[2-(3-Brom-thiophen-2-yl)-imidazo[1,2-a]pyridin-3-yl]-cyclohexyl-amin,
Essigsäure-2-methoxy-4-[3-(1,1,3,3-tetramethylbutylamino)-imidazo[1,2-a]pyrimidin-2-yl]-phenylester,
[2-(2-Chlor-4-fluor-phenyl)-imidazo[1,2-a]pyrimidin-3-yl]-(1,1,3,3-tetramethylbutyl)-amin,
(2-Anthracen-9-yl-imidazo[1,2-a]pyrazin-3-yl)-*tert*-butyl-amin,
*tert*-Butyl-(2-naphthalin-1-yl-imidazo[1,2-a]pyridin-3-yl)-amin,
N-Cyclohexyl-N-[2-(4,5-dimethyl-furan-2-yl)-imidazo[1,2-a]pyrimidin-3-yl]-acetamid oder
(1,1,3,3-Tetramethylbutyl)-[2-(3,4,5-trimethoxyphenyl)-imidazo[1,2-a]pyridin-3-yl]-amin.

Soweit die erfindungsgemäßen bicyclischen Imidazo-3-yl-amine optisch aktive Kohlenstoffatome enthalten, sind auch die Enantiomeren dieser Verbindungen und deren Mischungen Gegenstand der vorliegenden Erfindung.

Gegenstand der Erfindung sind außerdem Arzneimittel enthaltend als Wirkstoff mindestens ein bicyclisches Imidazo-3-yl-amin der allgemeinen Formel **I,** in der $R^1$ bis $R^3$, X und Y die oben angegebene Bedeutung haben,in Form der Base oder von pharmazeutisch akzeptablen Salzen, vorzugsweise der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure oder insbesondere der Salzsäure.

Überraschenderweise wurde dabei gefunden, daß die erfindungsgemäßen Verbindungen nicht nur potentielle Wirkstoffe für die im Stand der Technik genannten Indikationen sind, sondern auch analgetische Wirkung zeigen.

Besonders bevorzugt enthalten die erfindungsgemäßenen Arzneimittel als Wirkstoff mindestens ein bicyclisches Imidazo-3-yl-amin ausgewählt aus der Gruppe

(6-Isocyano-hexyl)-(2-pyridin-2-yl-imidazo[1,2-a]pyridin-3-yl)-amin,
(2-Furan-2-yl-imidazo[1,2-a]pyridin-3-yl)-(6-isocyanohexyl)-amin,
(2-Cyclohexyl-imidazo[1,2-a]pyrazin-3-yl)-(6-isocyanohexyl)-amin,
(2,6-Dimethyl-phenyl)-(2-furan-2-yl-imidazo[1,2-a]pyridin-3-yl)-amin,
(2-Furan-2-yl-imidazo[1,2-a]pyrazin-3-ylamino)-essigsäuremethylester,
(2-Cyclohexyl-imidazo[1,2-a]pyrimidin-3-ylamino)-essigsäuremethylester,
(2-Methyl-imidazo[1,2-a]pyrazin-3-ylamino)-essigsäuremethylester,
(2-Pyridin-4-yl-imidazo[1,2-a]pyrazin-3-yl)-(1,1,3,3-tetramethyl-butyl)-amin,
(2-Methyl-imidazo[1,2-a]pyrazin-3-yl)-(1,1,3,3-tetramethyl-butyl)-amin,
3-(3-tert-Butylamino-imidazo[1,2-a]pyridin-2-yl)-phenol,
Butyl-[2-(2,3-dichlor-phenyl)-imidazo[1,2-a]pyrazin-3-yl]-amin,
[(2-Phenyl-imidazo[1,2-a]pyridin-3-ylamino)-methyl]-phosphonsäurediethylester,
*tert*-Butyl-(2-*tert*-butyl-imidazo[1,2-a]pyridin-3-yl)-amin,
Butyl-(2-o-tolyl-imidazo[1,2-a]pyrimidin-3-yl)-amin, (2,6-Dimethyl-phenyl)-[2-(2-methoxy-phenyl)-imidazo[1,2-a]pyrazin-3-yl]-amin,
Butyl-(2-o-tolyl-imidazo[1,2-a]pyrimidin-3-yl)-amin, *tert*-Butyl-(2-pyridin-3-yl-imidazo[1,2-a]pyrimidin-3-yl)-amin,
*tert*-Butyl-(2-methyl-imidazo[1,2-a]pyridin-3-yl)-amin, [2-(1H-Pyrrol-2-yl)-imidazo[1,2-a]pyrimidin-3-yl]-(1,1,3,3-tetramethyl-butyl)-amin,
Cyclohexyl-(2-furan-2-yl-imidazo[1,2-a]pyridin-3-yl)-amin,
*tert*-Butyl-(2-pyridin-3-yl-imidazo[1,2-a]pyridin-3-yl)-amin,
*tert*-Butyl-(2-pyridin-2-yl-imidazo[1,2-a]pyridin-3-yl)-amin,
*tert*-Butyl-(2-thiophen-2-yl-imidazo[1,2-a]pyridin-3-yl)-amin,
Cyclohexyl-(2-methyl-imidazo[1,2-a]pyridin-3-yl)-amin, N-Cyclohexyl-N-[2-(5-methyl-furan-2-yl)-imidazo[1,2-a]pyridin-3-yl]-acetamid,
*tert*-Butyl-[2-(5-methylsulfanyl-thiophen-2-yl)-imidazo[1,2-a]pyrimidin-3-yl]-amin,
[2-(3-Brom-thiophen-2-yl)-imidazo[1,2-a]pyridin-3-yl]-cyclohexyl-amin,
Essigsäure-2-methoxy-4-[3-(1,1,3,3-tetramethylbutylamino)-imidazo[1,2-a]pyrimidin-2-yl]-phenylester,
[2-(2-Chlor-4-fluor-phenyl)-imidazo[1,2-a]pyrimidin-3-yl]-(1,1,3,3-tetramethylbutyl)-amin,
(2-Anthracen-9-yl-imidazo[1,2-a]pyrazin-3-yl)-*tert*butyl-amin,
*tert*-Butyl-(2-naphthalin-1-yl-imidazo[1,2-a]pyridin-3-yl)-amin,
N-Cyclohexyl-N-[2-(4,5-dimethyl-furan-2-yl)-imidazo[1,2-a]pyrimidin-3-yl]-acetamid oder (1,1,3,3-Tetramethylbutyl)-[2-(3,4,5-trimethoxyphenyl)-imidazo[1,2-a]pyridin-3-yl]-amin, oder der pharmazeutisch akzeptablen Salze dieser Verbindungen.

Besonders bevorzugt ist dabei die Verwendung der erfindungsgemäßen bicyclischen Imidazo-3-yl-amine zusammen mit einem oder mehreren Hilfsstoffen zur Herstellung eines Arzneimittels zur Bekämpfung von Schmerz.

Zur Herstellung entsprechender Arzneimittel werden neben mindestens einem erfindungsgemäßen Wirkstoff ein oder mehrere Hilfsstoffe, vorzugsweise Trägermaterialien, Füllstoffe, Lösungsmittel, Verdünnungsmittel, Farbstoffe und/oder Bindemittel eingesetzt. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich appliziert werden soll. Für orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Wirkstoffe in einem Depot, in gelöster Form oder in einem Pflaster, gegebenenfalls unter Zusatz von die Hautpenetration fördernden Mittel, sind geeignete perkutane Applikationszubereitungen. Oral oder perkutan anwendbare Zubereitungsformen können die erfindungsgemäßen Wirkstoffe verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung.

Die Synthese der erfindungsgemäßen Verbindungen erfolgt in der Weise, daß man Amidine mit der allgemeinen Formel **II,** insbesondere 2-Aminopyridin, 2-Aminopyrazin und 2-Aminopyrimidinderivate, die von Firmen wie beispielsweise Acros, Avocado, Aldrich, Fluka, Lancaster, Maybridge, Merck, Sigma oder TCI-Jp kommerziell angeboten werden, mit verschiedensten Ketonen oder vorzugsweise Aldehyden **III** und Isonitrilen **IV** in Gegenwart von 20%-iger Perchlorsäure gemäß einer Dreikomponentenraktion umsetzt. $R^1$ bis $R^3$, X und Y haben dabei die oben für Verbindungen der Formel **I** angegebene Bedeutung.

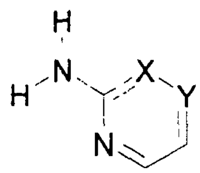

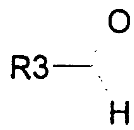

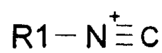

**II** **III** **IV**

Für einen problemlosen Ablauf der Reaktion ist es dabei wesentlich, daß die Ausgangsverbindungen nacheinander in der Reihenfolge Amidin **II**, Aldehyd **III** und Isonitril **IV** zugegeben werden. Vorzugsweise werden die Reaktionen in Dichlormethan bei einer Temperatur von vorzugsweise 0°C bis 40°C, insbesondere bei einer Temperatur von 10°C bis 20°C durchgeführt.

Zur Herstellung der erfindungsgemäßen Verbindungen, in denen $R^2$ nicht Wasserstoff bedeutet, werden die in der zuvor beschriebenen Reaktion entstehenden Verbindungen **Ia,** die vorzugsweise zunächst in THF gelöst wurden, je nach gewünschtem Endprodukt mit einer Verbindung $R^2$Hal, wobei Hal für Brom, Iod oder insbesondere Chlor steht, beispielsweise einem gegebenenfalls substituierten Alkyl-, Aryl- oder Säurechlorid, oder einem gegebenenfalls substituierten Isocyanat $R^e$NCO in Gegenwart eines Morpholin-Harzes (z.B. Polystyrol-Morpholin der Firma Argonaut) in Dichlormethan innerhalb von 2 bis 24 Stunden bei Temperaturen zwischen 10°C und 40°C gemäß dem folgenden Reaktionsschema umgesetzt:

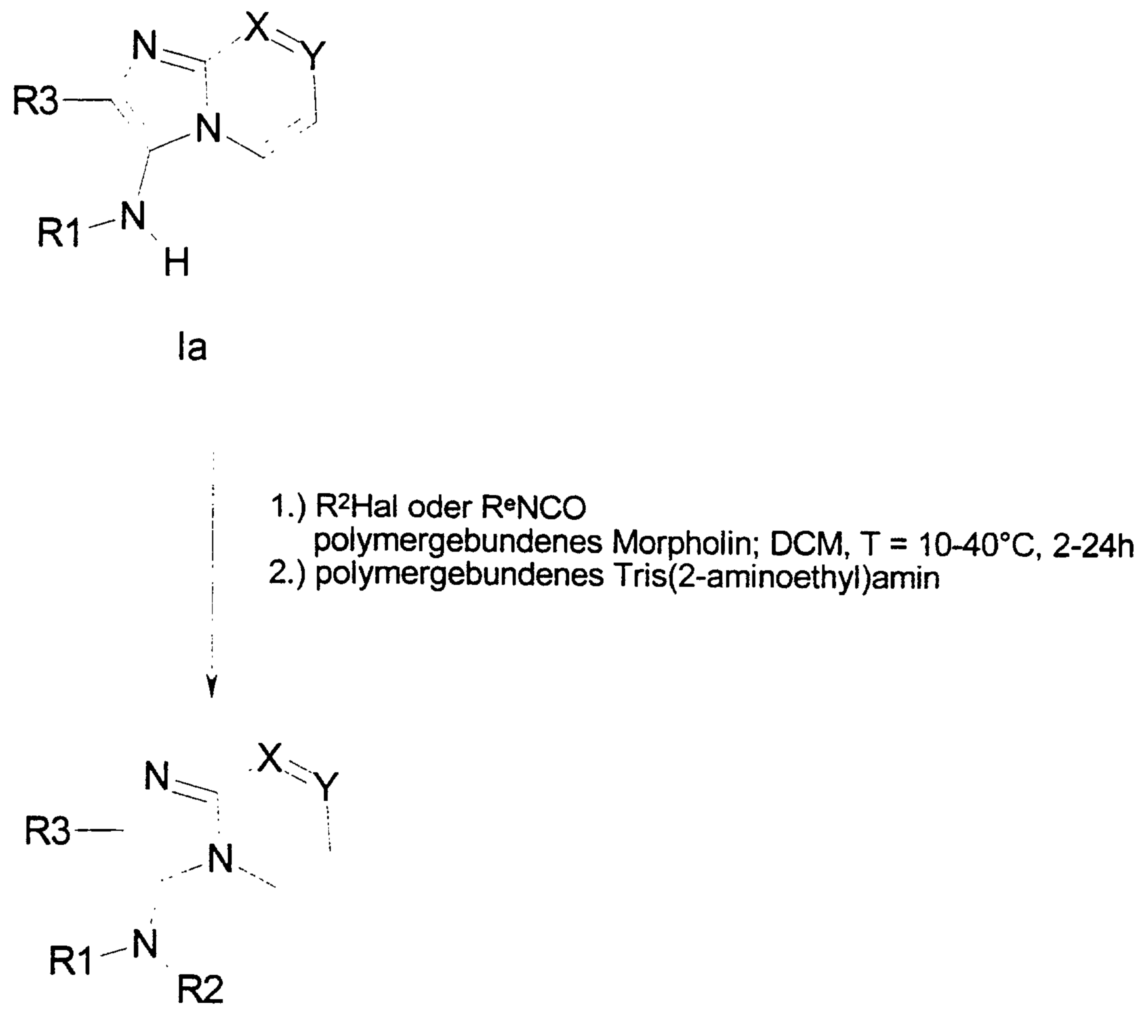


Ia

I

Die überschüssigen Reagentien werden anschließend durch Filtration über eine Schicht mit polymergebundenem Tris(2-aminoethyl)amin (Hersteller: Novabiochem) oder 3-(3-Mercaptophenyl)propanamidomethylpolystyrol aus dem Reaktionsgemisch entfernt und das Filtrat vorzugsweise in einer Vakuumzentrifuge aufkonzentriert. Das gesamte Verfahren läßt sich ohne weiteres auch in einer automatisierten Syntheseanlage durchführen.

Die Verbindungen der Formel **I** lassen sich mit physiologisch verträglichen Säuren, vorzugsweise Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure und insbesondere Salzsäure, in der an sich bekannten Weise in ihre Salze überführen. Vorzugsweise wird die Salzbildung in einem Lösungsmittel, vorzugsweise Diethylether, Diisopropylether, Essigsäurealkylester, Aceton oder 2-Butanon oder einem Gemisch dieser Lösungsmittel durchgeführt. Zur Herstellung der Hydrochloride eignet sich alternativ auch Trimethylsilan in wässriger Lösung.

**Beispiele:**

Die folgenden Beispiele sollen die Erfindung erläutern, ohne sie darauf zu beschränken.

**Allgemeine Vorschrift 1 (Automatische Synthese)**

Die Synthese der erfolgte auf einer automatischen Anlage der Firma Zymark nach folgender allgemeiner Synthesevorschrift:

Ein Rundbodenröhrchen aus Glas (Durchmesser 16 mm, Länge 125 mm) mit Gewinde wurde manuell mit

einem Rührer versehen und auf der Capper-Station mit einem Schraubdeckel mit Septum verschlossen. Das Röhrchen wurde von Roboter 1 in den auf 15°C temperierten Reaktorblock gestellt. Roboter 2 pipettierte nacheinander folgende Reagenzien hinzu:

1.) 1 ml einer 0,1 M Amidin-Lösung + 20% $HClO_4$ in Dichlormethan
2.) 0,5 ml einer 0,3 M Aldehyd-Lösung in Dichlormethan
3.) 0,575 ml einer 0,2 M Isonitril-Lösung in Dichlormethan

Das Reaktionsgemisch wurde bei 15°C in einem der Rührblöcke 660 min lang gerührt. Danach wurde die Reaktionslösung an der Filtrations-Station abfiltriert. Das Röhrchen wurde dabei zweimal mit je 1 ml Dichlormethan und 200 µl Wasser gespült.

Das Rack mit den Röhrchen wurde anschließend manuell auf die Aufarbeitungsanlage gestellt. Dort wurde das Reaktionsgemisch auf einem Vortexer mit 3 ml einer 10%igen NaCl-Lösung und 1,5 ml Dichlormethan versetzt. Im Spin-Reaktor wurde zehn Minuten lang gründlich gemischt und durch die langsame Abnahme der Drehbewegung eine deutliche Phasengrenze ausgebildet. Diese Phasengrenze wurde optisch detektiert und die organische Phase abpipettiert. Im nächsten Schritt wurde das Reaktionsgemisch erneut mit 1,5 ml Dichlormethan versetzt. Die Lösung wurde geschüttelt, zentrifugiert und die organische Phase abpipettiert. Die vereinigten organischen Phasen wurden über 2,4 g $MgSO_4$ (granuliert) getrocknet. Das Lösungsmittel wurde in einer Vakuumzentrifuge entfernt.

**Allgemeine Vorschrift 2 (Manuelle Synthese)**

(Äquivalente bedeuten Moläquivalente bezogen auf das eingesetzte Isonitril):

In einem geeigneten Reaktionsgefäß wurden zunächst 1,15 Äquivalente des heterocyclischen Amins in Dichlormethan (2 ml je mmol eingesetztem Isonitril) suspendiert bzw. gelöst. Hierzu wurden nacheinander 1,5 Äquivalente Aldehyd, ein Äquivalent Isonitril und schließlich wäßrige Perchlorsäurelösung (20 m%; 0,098 ml je mmol eingesetztem Isonitril) zugegeben und der Ansatz für zwanzig Stunden bei Raumtemperatur gerührt.
Zur Aufarbeitung wurden gesättigte Natriumchloridlösung (ca. 5 ml je mmol eingesetztem Isonitril) und Dichlormethan (ca. 4 ml je mmol eingesetztem Isonitril) zugegeben, die Phasen getrennt und die organische Phase noch zweimal mit Dichlormethan (je ca. 2 ml je mmol eingesetztem Isonitril) extrahiert. Die vereinigten organischen Phasen wurden nacheinander mit Pufferlösung (pH10; ca. 2 ml je mmol eingesetztem Isonitril) und ges. Natriumchloridlösung (ca. 2 ml je mmol eingesetztem Isonitril) gewaschen, über Natriumsulfat getrocknet, filtriert, am Rotationsverdampfer im Vakuum eingeengt und im Ölpumpenvakuum von Lösungsmittelresten befreit.

Die eingesetzten Chemikalien und Lösungsmittel wurden kommerziell erworben. Jede Substanz wurde mit ESI-MS und/oder NMR analysiert.

**Allgemeine Vorschrift 3 (Umsetzung mit Acetylchlorid)**

Das nach der allgemeinen Vorschrift 1 erhaltene Produkt wurde in Dichlormethan gelöst, mit 4 Moläquivalenten Acetylchlorid versetzt und vier Stunden bei 18 °C gerührt. Das überschüssige Acetylchlorid und das Lösungsmittel wurden bei 40-60°C im Vakuum entfernt. Jede Substanz wurde mit ESI-MS analysiert.

**Beispiel 1**

(6-Isocyano-hexyl)-(2-pyridin-2-yl-imidazo[1,2-**a**]pyridin-3-yl)-amin (**1**)

Verbindung **1** wurde gemäß der allgemeinen Vorschrift 1 aus 1.0 ml 2-Aminopyridin-Lösung (0.1 M, DCM), 0.575 ml 1,6-Diisocyanhexan-Lösung (0.2 M, DCM), 0.500 ml Pyridin-2-carbaldehyd-Lösung (0.3 M, DCM) und 10 µl Perchlorsäure (w= 20%) dargestellt.
Berechnete Masse 321,43; gefundene Masse M-H = 320,4 (ESI-MS)

**Beispiel 2**

(2-Furan-2-yl-imidazo[1,2-a]pyridin-3-yl)-(6-isocyanohexyl)-amin (**2**)

Verbindung **2** wurde gemäß der allgemeinen Vorschrift 1 aus 1.0 ml 2-Aminopyridin-Lösung (0.1 M, DCM), 0.575 ml 1,6-Diisocyanhexan-Lösung (0.2 M, DCM), 0.500 ml Furfural-Lösung (0.3 M, DCM) und 10 µl Perchlorsäure (w= 20%) dargestellt.

Berechnete Masse 310,40; gefundene Masse M-H = 309,4 (ESI-MS)

**Beispiel 3**

(2-Cyclohexyl-imidazo[1,2-a]pyrazin-3-yl)-(6-isocyanohexyl)-amin (**3**)

Verbindung **3** wurde gemäß der allgemeinen Vorschrift 1 aus 1.0 ml Aminopyrazin-Lösung (0.1 M, DCM), 0.575 ml 1,6-Diisocyanhexan-Lösung (0.2 M, DCM), 0.500 ml Cyclohexancarbaldehyd-Lösung (0.3 M, DCM) und 10 µl Perchlorsäure (w= 20%) dargestellt.
Berechnete Masse 327,48; gefundene Masse M-H = 326,5 (ESI-MS)

**Beispiel 4**

(2,6-Dimethyl-phenyl)-(2-furan-2-yl-imidazo[1,2-a]pyridin-3-yl)-amin (**4**)

Verbindung **4** wurde gemäß der allgemeinen Vorschrift 1 aus 1.0 ml 2-Aminopyridin-Lösung (0.1 M, DCM), 0.575 ml 2,6-Dimethylphenylisonitril-Lösung (0.2 M, DCM), 0.500 ml Furfural-Lösung (0.3 M, DCM) und 10 µl Perchlorsäure (w= 20%) dargestellt.
Berechnete Masse 303,37; gefundene Masse = 304,4 (ESI-MS)

**Beispiel 5**

(2-Furan-2-yl-imidazo[1,2-a]pyrazin-3-ylamino)-essigsäuremethylester (**5**)

Verbindung **5** wurde gemäß der allgemeinen Vorschrift 1 aus 1.0 ml Aminopyrazin-Lösung (0.1 M, DCM), 0.575 ml Isocyanoessigsäuremethylester-Lösung (0.2 M, DCM), 0.500 ml Furfural-Lösung (0.3 M, DCM) und 10 µl Perchlorsäure (w= 20%) dargestellt.
Berechnete Masse 272,27; gefundene Masse = 273,4 (ESI-MS)

**Beispiel 6**

(2-Cyclohexyl-imidazo[1,2-a]pyrimidin-3-ylamino)-essigsäuremethylester (**6**)

Verbindung **6** wurde gemäß der allgemeinen Vorschrift 1 aus 1.0 ml 2-Aminopyrimidin-Lösung (0.1 M, DCM), 0.575 ml Isocyanoessigsäuremethylester-Lösung (0.2 M, DCM), 0.500 ml Cyclohexylcarbaldehyd-Lösung (0.3 M, DCM) und 10 µl Perchlorsäure (w= 20%) dargestellt.
Berechnete Masse 288,35; gefundene Masse = 289,4 (ESI-MS)

**Beispiel 7**

(2-Methyl-imidazo[1,2-a]pyrazin-3-ylamino)-essigsäuremethylester

Verbindung **7** wurde gemäß der allgemeinen Vorschrift 1 aus 1.0 ml Aminopyrazin-Lösung (0.1 M, DCM), 0.575 ml Isocyanoessigsäuremethylester-Lösung (0.2 M, DCM), 0.500 ml Acetaldehyd-Lösung (0.3 M, DCM) und 10 µl Perchlorsäure (w= 20%) dargestellt.
Berechnete Masse 220,23; gefundene Masse = 221,3 (ESI-MS)

**Beispiel 8**

(2-Pyridin-4-yl-imidazo[1,2-a]pyrazin-3-yl)-(1,1,3,3-tetramethyl-butyl)-amin (**8**)

Verbindung **8** wurde gemäß der allgemeinen Vorschrift 1 aus 1.0 ml Aminopyrazin-Lösung (0.1 M, DCM), 0.575 ml 1,1,3,3-Tetramethylbutylisocyanid (0.2 M, DCM), 0.500 ml Pyridin-4-carbaldehyd-Lösung (0.3 M, DCM) und 10 µl Perchlorsäure (w= 20%) dargestellt.
Berechnete Masse 323,44; gefundene Masse = 324,4 (ESI-MS)

**Beispiel 9**

(2-Methyl-imidazo[1,2-a]pyrazin-3-yl)-(1,1,3,3-tetramethyl-butyl)-amin

Verbindung **9** wurde gemäß der allgemeinen Vorschrift 1 aus 1.0 ml Aminopyrazin-Lösung (0.1 M, DCM), 0.575 ml 1,1,3,3-Tetramethylbutylisocyanid (0.2 M, DCM), 0.500 ml Acetaldehyd-Lösung (0.3 M, DCM) und 10 µl Perchlorsäure (w= 20%) dargestellt.
Berechnete Masse 260,39; gefundene Masse = 261,4 (ESI-MS)

**Beispiel 10**

3-(3-*tert*-Butylamino-imidazo[1,2-a]pyridin-2-yl)-phenol(**10**)

Verbindung 10 wurde gemäß der allgemeinen Vorschrift 1 aus 1.0 ml 2-Aminopyridin-Lösung (0.1 M, DCM), 0.575 ml *tert*.-Butylisonitril-Lösung (0.2 M, DCM), 0.500 ml 3-Hydroxybenzaldehyd-Lösung (0.3 M, DCM) und 10 µl Perchlorsäure (w= 20%) dargestellt.
Berechnete Masse 281, 36; gefundene Masse = 282,3 (ESI-MS)

**Beispiel 11**

Butyl-[2-(2,3-dichlor-phenyl)-imidazo[1,2-a]pyrazin-3-yl]-amin (11)

Verbindung **11** wurde gemäß der allgemeinen Vorschrift 1 aus 1.0 ml Aminopyrazin-Lösung (0.1 M, DCM), 0.575 ml n-Butylisonitril-Lösung (0.2 M, DCM), 0.500 ml 2,3-Dichlorbenzaldehyd-Lösung (0.3 M, DCM) und 10 µl Perchlorsäure (w= 20%) dargestellt.
Berechnete Masse 335,24; gefundene Masse = 335,4 (ESI-MS)

**Beispiel 12**

[(2-Phenyl-imidazo[1,2-a]pyridin-3-ylamino)-methyl]-phosphonsäurediethylester (**12**)

Verbindung 12 wurde gemäß der allgemeinen Vorschrift 2 aus 2-Aminopyridin Diethylisocyanomethylphosphat, Benzaldehyd und Perchlorsäure (w= 20%) dargestellt.
Die Struktur wurde durch NMR-Spektroskopie bestätigt.

**Beispiel 13**

*tert*-Butyl-(2-*tert*-butyl-imidazo[1,2-a]pyridin-3-yl)-amin (13)

Verbindung 13 wurde gemäß der allgemeinen Vorschrift 2 aus 2-Aminopyridin, *tert*.-Butylisonitril, Pivaldehyd und Perchlorsäure dargestellt.
Die Struktur wurde durch NMR-Spektroskopie bestätigt.

**Beispiel 14**

Butyl-(2-*o*-tolyl-imidazo[1,2-a]pyrimidin-3-yl)-amin (**14**)

Verbindung **14** wurde gemäß der allgemeinen Vorschrift aus 1.0 ml 2-Aminopyrimidin-Lösung (0.1 M, DCM), 0.575 ml n-Butylisonitril-Lösung (0.2 M, DCM), 0.500 ml 2-Methylbenzaldehyd-Lösung (0.3 M, DCM) und 10 µl Perchlorsäure (w= 20%) dargestellt.
Berechnete Masse 280,38; gefundene Masse = 281,3 (ESI-MS)

**Beispiel 15**

(2,6-Dimethyl-phenyl)-[2-(2-methoxy-phenyl)-imidazo[1,2-a]pyrazin-3-yl]-amin (**15**)

Verbindung **15** wurde gemäß der allgemeinen Vorschrift 1 aus 1.0 ml Aminopyrazin-Lösung (0.1 M, DCM), 0.575 ml 2,6-Dimethylphenylisocyanid-Lösung (0.2 M, DCM), 0.500 ml 2-Methoxybenzaldehyd-Lösung (0.3 M, DCM)

und 10 µl Perchlorsäure (w= 20%) dargestellt.
Berechnete Masse 344,42; gefundene Masse = 345,4 (ESI-MS)

**Beispiel 16**

Butyl-(2-*o*-tolyl-imidazo[1,2-a]pyrimidin-3-yl)-amin (**16**)

Verbindung **16** wurde gemäß der allgemeinen Vorschrift 1 aus 1.0 ml 2-Aminopyrimidin-Lösung (0.1 M, DCM), 0.575 n-Butylisonitril-Lösung (0.2 M, DCM), 0.500 ml 2-Methylbenzaldehyd-Lösung (0.3 M, DCM) und 10 µl Perchlorsäure (w= 20%) dargestellt.
Berechnete Masse 280,38; gefundene Masse = 281,3 (ESI-MS)

**Beispiel 17**

*tert*-Butyl-(2-pyridin-3-yl-imidazo[1,2-a]pyrimidin-3-yl)-amin (**17**)

Verbindung **17** wurde gemäß der allgemeinen Vorschrift 1 aus 1.0 ml 2-Aminopyrimidin-Lösung (0.1 M, DCM), 0.575 ml *tert*.-Butylisonitril-Lösung (0.2 M, DCM), 0.500 ml Pyridin-3-carbaldehyd-Lösung (0.3 M, DCM) und 10 µl Perchlorsäure (w= 20%) dargestellt.
Berechnete Masse 267,34; gefundene Masse = 268,3 (ESI-MS)

**Beispiel 18**

*tert*-Butyl-(2-methyl-imidazo[1,2-a]pyridin-3-yl)-amin (**18**)

Verbindung 18 wurde gemäß der allgemeinen Vorschrift 1 aus 1.0 ml 2-Aminopyridin-Lösung (0.1 M, DCM), 0.575 ml *tert*-Butylisonitril-Lösung (0.2 M, DCM), 0.500 ml Acetaldehyd-Lösung (0.3 M, DCM) und 10 µl Perchlorsäure (w= 20%) dargestellt.
Berechnete Masse 203,29; gefundene Masse = 204,3 (ESI-MS)

**Beispiel 19**

[2-(1*H*-Pyrrol-2-yl)-imidazo[1,2-a]pyrimidin-3-yl]-(1,1,3,3-tetramethyl-butyl)-amin (**19**)

Verbindung **19** wurde gemäß der allgemeinen Vorschrift 1 aus 1.0 ml 2-Aminopyrimidin-Lösung (0.1 M, DCM), 0.575 ml 1,1,3,3-Tetramethylbutyl-isocyanid-Lösung (0.2 M, DCM), 0.500 ml Pyrrol-2-carbaldehyd-Lösung (0.3 M, DCM) und 10 µl Perchlorsäure (w= 20%) dargestellt.
Berechnete Masse 311,43; gefundene Masse = 312,4 (ESI-MS)

**Beispiel 20**

Cyclohexyl-(2-furan-2-yl-imidazo[1,2-a]pyridin-3-yl)-amin (**20**)

Verbindung **20** wurde gemäß der allgemeinen Vorschrift 2 aus 2-Aminopyridin, Cyclohexylisonitril, Furfural und Perchlorsäure dargestellt.
Die Struktur wurde durch NMR-Spektroskopie bestätigt.

**Beispiel 21**

*tert*-Butyl-(2-pyridin-3-yl-imidazo[1,2-a]pyridin-3-yl)-amin (**21**)

Verbindung **21** wurde gemäß der allgemeinen Vorschrift 2 aus 2-Aminopyridin, *tert*-Butylisonitril, Nicotinaldehyd und Perchlorsäure dargestellt.
Die Struktur wurde durch NMR-Spektroskopie bestätigt.

**Beispiel 22**

*tert*-Butyl-(2-pyridin-2-yl-imidazo[1,2-a]pyridin-3-yl)-amin (**22**)

Verbindung **22** wurde gemäß der allgemeinen Vorschrift 2 aus 2-Aminopyridin, *tert*-Butylisonitril, 2-Pyridylcarbaldehyd und Perchlorsäure dargestellt.
Die Struktur wurde durch NMR-Spektroskopie bestätigt.

**Beispiel 23**

*tert*-Butyl-(2-thiophen-2-yl-imidazo[1,2-a]pyridin-3-yl)-amin (**23**)

Verbindung **23** wurde gemäß der allgemeinen Vorschrift 2 aus 2-Aminopyridin, *tert*-Butylisonitril, Thiophen-2-carbaldehyd und Perchlorsäure dargestellt.
Die Struktur wurde durch NMR-Spektroskopie bestätigt.

**Beispiel 24**

Cyclohexyl-(2-methyl-imidazo[1,2-a]pyridin-3-yl)-amin (**24**)

Verbindung **24** wurde gemäß der allgemeinen Vorschrift 2 aus 2-Aminopyridin, Cyclohexylisonitril, Acetaldehyd und Perchlorsäure dargestellt.
Die Struktur wurde durch NMR-Spektroskopie bestätigt.

**Beispiel 25**

N-Cyclohexyl-N-[2-(5-methyl-furan-2-yl)-imidazo[1,2-a]pyridin-3-yl]-acetamid (**25**)

Verbindung **25** wurde durch Umsetzung des gemäß der allgemeinen Vorschrift 1 aus 1.0 ml 2-Aminopyridin-Lösung (0.1 M, DCM), 0.575 ml Cyclohexylisocyanid-Lösung (0.2 M, DCM), 0.500 ml 5-Methylfurfural-Lösung (0.3 M, DCM) und 10 µl Perchlorsäure (w= 20%) erhaltenen Produkts mit Acetylchlorid gemäß der allgemeinen Vorschrift 3 dargestellt.
Berechnete Masse 337,4; gefundene Masse 338,5; M-Acetyl 296, 5 (ESI-MS)

**Beispiel 26**

*tert*-Butyl-[2-(5-methylsulfanyl-thiophen-2-yl)-imidazo[1,2-a]pyrimidin-3-yl]-amin (**26**)

Verbindung **26** wurde gemäß der allgemeinen Vorschrift 1 aus 1.0 ml 2-Aminopyrimidin-Lösung (0.1 M, DCM), 0.575 ml *tert*-Butylisonitrilisocyanid-Lösung (0.2 M, DCM), 0.500 ml 5-Methylsulfanyl-thiophen-2-carbaldehyd-Lösung (0.3 M, DCM) und 10 µl Perchlorsäure (w= 20%) dargestellt. Berechnete Masse 318,5; gefundene Masse 319,2 (ESI-MS)

**Beispiel 27**

[2-(3-Brom-thiophen-2-yl)-imidazo[1,2-a]pyridin-3-yl]-cyclohexyl-amin (**27**)

Verbindung **27** wurde gemäß der allgemeinen Vorschrift 1 aus 1.0 ml 2-Aminopyridin-Lösung (0.1 M, DCM), 0.575 ml Cyclohexylisocyanid-Lösung (0.2 M, DCM), 0.500 3-Bromthiophen-2-carbaldehyd-Lösung (0.3 M, DCM) und 10 µl Perchlorsäure (w= 20%) dargestellt. Berechnete Masse 376,3; gefundene Masse 376,4/378,3 (ESI-MS)

**Beispiel 28**

Essigsäure-2-methoxy-4-[3-(1,1,3,3-tetramethylbutylamino)-imidazo[1,2-a]pyrimidin-2-yl]-phenylester (**28**)

Verbindung **28** wurde gemäß der allgemeinen Vorschrift 1 aus 1.0 ml 2-Aminopyrimidin-Lösung (0.1 M, DCM), 0.575 ml 1,1,3,3-Tetramethylbutylisocyanid-Lösung (0.2 M, DCM), 0.500 Essigsäure-4-formyl-2-methoxy-phenylester-Lösung (0.3 M, DCM) und 10 µl Perchlorsäure (w= 20%) dargestellt. Berechnete Masse 410,5; gefundene Masse 411,3

(ESI-MS)

**Beispiel 29**

[2-(2-Chlor-4-fluor-phenyl)-imidazo[1,2-a]pyrimidin-3-yl]-(1,1,3,3-tetramethylbutyl)-amin (**29**)

Verbindung **29** wurde gemäß der allgemeinen Vorschrift 1 aus 1.0 ml 2-Aminopyrimidin-Lösung (0.1 M, DCM), 0.575 ml 1,1,3,3-Tetramethylbutylisocyanid-Lösung (0.2 M, DCM), 0.500 2-Chlor-4-fluorbenzaldehyd-Lösung (0.3 M, DCM) und 10 µl Perchlorsäure (w= 20%) dargestellt. Berechnete Masse 374,9; gefundene Masse 375,3 (ESI-MS)

**Beispiel 30**

(2-Anthracen-9-yl-imidazo[1,2-a]pyrazin-3-yl)-*tert*-butyl-amin (**30**)

Verbindung **30** wurde gemäß der allgemeinen Vorschrift 1 aus 1.0 ml 2-Aminopyrazin-Lösung (0.1 M, DCM), 0.575 ml *tert*-Butylisocyanid-Lösung (0.2 M, DCM), 0.500 Anthracen-9-carbaldehyd-Lösung (0.3 M, DCM) und 10 µl Perchlorsäure (w= 20%) dargestellt. Berechnete Masse 366,5; gefundene Masse 367,3 (ESI-MS)

**Beispiel 31**

*tert*-Butyl-(2-naphthalin-1-yl-imidazo[1,2-a]pyridin-3-yl)-amin (**31**)

Verbindung **31** wurde gemäß der allgemeinen Vorschrift 1 aus 1.0 ml 2-Aminopyridin-Lösung (0.1 M, DCM), 0.575 ml *tert*-Butylisocyanid-Lösung (0.2 M, DCM), 0.500 Naphtalin-1-carbaldehyd-Lösung (0.3 M, DCM) und 10 µl Perchlorsäure (w= 20%) dargestellt. Berechnete Masse 315,4; gefundene Masse 316,3 (ESI-MS)

**Beispiel 32**

N-Cyclohexyl-N-[2-(4,5-dimethyl-furan-2-yl)-imidazo[1,2-a]pyrimidin-3-yl]-acetamid (**32**)

Verbindung **32** wurde durch Umsetzung des gemäß der allgemeinen Vorschrift 1 aus 1.0 ml 2-Aminopyrimidin-Lösung (0.1 M, DCM), 0.575 ml Cyclohexylisocyanid-Lösung (0.2 M, DCM), 0.500 4,5-Dimethylfurfural-Lösung (0.3 M, DCM) und 10 µl Perchlorsäure (w= 20%) erhaltenen Produkts mit Acetylchlorid gemäß der allgemeinen Vorschrift 3 dargestellt.
Berechnete Masse 352,4; gefundene Masse 353,4 (ESI-MS)

**Beispiel 33**

(1,1,3,3-Tetramethylbutyl)-[2-(3,4,5-trimethoxyphenyl)-imidazo[1,2-a]pyridin-3-yl]-amin (**33**)

Verbindung **33** wurde gemäß der allgemeinen Vorschrift 1 aus 1.0 ml 2-Aminopyridin-Lösung (0.1 M, DCM), 0.575 ml 1,1,3,3-Tetramethylbutylisocyanid-Lösung (0.2 M, DCM), 0.500 3,4,5-Trimethoxybenzaldehyd-Lösung (0.3 M, DCM) und 10 µl Perchlorsäure (w= 20%) dargestellt.
Berechnete Masse 411,5; gefundene Masse 412,3 (ESI-MS)

**Analgesieprüfung im Writhing-Test an der Maus**

Die Untersuchung auf analgetische Wirksamkeit wurde im Phenylchinon-induzierten Writhing an der Maus (modifiziert nach I.C. Hendershot, J. Forsaith, J. Pharmacol. Exp. Ther. 125, 237-240 (1959)) durchgeführt. Dazu wurden männliche NMRI-Mäuse im Gewicht von 25-30 g verwendet. Gruppen von 10 Tieren pro Substanzdosis erhielten 10 Minuten nach intravenöser oder subcutaner Gabe der Prüfsubstanzen 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige gesetzt. Mittels eines Drucktastenzähler wurde die Anzahl der Schmerzinduzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die nur physiologische Kochsalzlösung erhalten. Die Substanzen wurden in der Standarddosierung von 10 mg/kg intravenös oder 21,5 mg/kg subcutan getestet. Die prozentuale Hemmung (%Hemmung) der Writhingreaktion durch eine Substanz wurde nach folgender

Formel berechnet:

$$\% \text{ Hemmung} = 100 - \frac{\text{Writhingreaktionen der behandelten Tiere}}{\text{Writhingreaktionen der Kontrolltiere}} * 100$$

Die untersuchten erfindungsgemäßen Verbindungen zeigten eine analgetische Wirkung. Die Ergebnisse sind in der nachfolgenden Tabelle zusammengefaßt.

Tabelle:

| Analgesieprüfung im Writhing-Test an der Maus | | | |
|---|---|---|---|
| Beispiel | %Hemmung der Writhingreaktion bei 21,5 mg/kg subcutan | %Hemmung der Writhingreaktion bei 10 mg/kg intravenös | |
| 12 | | 90 | |
| 13 | | 86 bei 2,15 mg/kg | |
| 20 | | 43 | |
| 21 | 80 | | |
| 22 | | 53 | |
| 23 | | 62 | |
| 24 | | 56 | |

## Patentansprüche

1. Bicyclische Imidazo-3-yl-amine der allgemeinen Formel I,

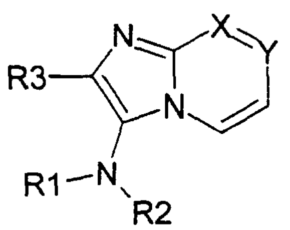

I

worin

X und Y CH oder N bedeuten mit der Maßgabe, daß X und Y nicht gleichzeitig N bedeuten,

$R^1$ *tert*-Butyl, $(CH_2)_nCN$ mit n = 4 ,5 oder 6, gegebenenfalls substituiertes Phenyl, $C_4$-$C_8$-Cycloalkyl, $CH_2CH_2R$ (R = 4-Morpholino), 1, 1, 3, 3-Tetramethylbutyl oder $CH_2R^a$, wobei $R^a$ für Wasserstoff, OH, $C_1$-$C_8$-Alkyl (verzweigt oder unverzweigt), gegebenenfalls substituiertes Phenyl, CO(OR') (mit R' = unverzweigtes $C_1$-$C_4$-Alkyl oder verzweigtes $C_1$-$C_5$-Alkyl), $PO(OR')_2$ (mit R' = unverzweigtes $C_1$-$C_4$-Alkyl oder verzweigtes $C_1$-$C_5$-Alkyl) oder $Si(R^xR^yR^z)$ (mit $R^x$, $R^y$, und $R^z$ jeweils unabhängig voneinander $C_1$-$C_4$-Alkyl(verzweigt oder unverzweigt), $C_4$-$C_8$-Cycloalkyl oder Phenyl) steht, bedeutet,

$R^2$ Wasserstoff, $COR^b$, wobei $R^b$ für $C_1$-$C_4$-Alkyl(verzweigt oder unverzweigt)oder $C_3$-$C_8$-Cycloalkyl steht, $CH_2CH_2CO(OR^c)$, wobei $R^c$ für $C_1$-$C_4$-Alkyl (verzweigt oder unverzweigt), Adamantyl, gegebenenfalls substituiertes Phenyl, gegebenenfalls substituiertes 1-Naphtyl oder 2-Naphtyl oder jeweils gegebenenfalls substituiertes 2-Pyridyl, 3-Pyridyl, 4-Pyridyl, Thiazolyl oder Furoyl steht, $CH_2$Phenyl, $CH_2CH_2R^d$, wobei $R^d$ für gegebenenfalls substituiertes Phenyl steht, oder $CONHR^e$, wobei $R^e$ für $C_1$-$C_8$-Alkyl (verzweigt oder unverzweigt), $C_3$-$C_8$-Cycloalkyl oder gegebenenfalls substiuiertes Phenyl steht, bedeutet

$R^3$ Methyl, Ethyl, *tert*-Butyl, $C_3$-$C_8$-Cycloalkyl,Phenyl, gegebenenfalls einfach substituiert in 3-, 5- oder 6-Position oder gegebenenfalls mehrfach substituiert in 4-Position und zusätzlich in 2- und/oder 3- und/oder 5- und/oder 6-Position, Phenoxy, gegebenenfalls substituiertes Naphtyl, gegebenenfalls substituiertes Pyrrol, gegebenenfalls substituiertes Pyridyl, gegebenenfalls substituiertes Furan, gegebenenfalls substituiertes Thiophen, gegebenenfalls substituiertes Anthracen, gegebenenfalls substituiertes Phenanthren oder gegebenenfalls substituiertes Chinolin bedeutet,

mit der Maßgabe, daß $R^3$ nicht n-Propyl, Cyclohexyl, unsubstituiertes Phenyl oder in 3-Position mit einer Carbonsäureamid-Gruppe monosubstituiertes Phenyl bedeutet, wenn $R^1$ *tert*-Butyl, n-Propyl, n-Butyl, 1,1,3,3-Tetramethylbutyl, Cyclohexyl, $CH_2CH_2R$ (R = 4-Morpholino), monosubstituiertes Phenyl, 2,6,-Dimethylphenyl oder Benzyl bedeutet und gleichzeitig $R^2$ Wasserstoff oder -CO(Methyl) bedeutet, und daß $R^2$ nicht Wasserstoff bedeutet, wenn gleichzeitig $R^1$ Benzyl und $R^3$ Methyl bedeutet oder gleichzeitig $R^1$ $CH_2C(O)$*tert*-Butyl und $R^3$ unsubstituiertes Phenyl bedeutet, in Form der Basen oder von pharmazeutisch akzeptablen Salzen.

**2.** Bicyclische Imidazo-3-yl-amine nach Anspruch 1, **dadurch gekennzeichnet, daß** $R^2$ Wasserstoff bedeutet,
$R^1$ ausgewählt ist aus der Gruppe $(CH_2)_nCN$ mit n = 4, 5 oder 6, Cyclohexyl, $CH_2CO$(OMethyl), 2,6-Dimethylphenyl, 1,1,3,3,-Tetramethylbutyl, tert-Butyl oder n-Butyl und
$R^3$ ausgewählt ist aus der Gruppe 2-Pyridyl, 3-Pyridyl, 2-Furanyl, 2-Pyrroyl, Methyl, *tert*-Butyl, 3-Hydroxyphenyl, 3,4-Dimethoxyphenyl, 2,3-Dichlorphenyl, 2,4-Dichlorphenyl, 2-Methoxyphenyl, 2,3-Dimethoxyphenyl, 3-Bromphenyl, 4-Brom-2-fluorphenyl, 5-Brom-2-fluorphenyl, 3-Brom-4-fluorphenyl, 3-Chlorphenyl, 3,4-Dichlorphenyl, 3-Fluorphenyl, 3-Methylphenyl, 3-Phenoxyphenyl, 3-(4-Chlorphenoxy)phenyl, 2-Chlor-4-fluorphenyl, 2-Chlor-6-fluorphenyl, 2,4-Dimethylphenyl, 2,5-Dimethylphenyl, 2-Bromphenyl, 2-Fluorphenyl, 2-(Trifluormethyl)-phenyl.

**3.** Bicyclische Imidazo-3-yl-amine gemäß Anspruch 1 oder 2, **dadurch gekennzeichnet, daß** es sich um

(6-Isocyano-hexyl)-(2-pyridin-2-yl-imidazo[1,2-a]pyridin-3-yl)-amin,
(2-Furan-2-yl-imidazo[1,2-a]pyridin-3-yl)-(6-isocyano-hexyl)-amin,
(2-Cyclohexyl-imidazo[1,2-a]pyrazin-3-yl)-(6-isocyano-hexyl)-amin,
(2,6-Dimethyl-phenyl)-(2-furan-2-yl-imidazo[1,2-a]pyridin-3-yl)-amin,
(2-Furan-2-yl-imidazo[1,2-a]pyrazin-3-ylamino)-essigsäuremethylester,
(2-Cyclohexyl-imidazo[1,2-a]pyrimidin-3-ylamino)-essigsäuremethylester,
(2-Methyl-imidazo[1,2-a]pyrazin-3-ylamino)-essigsäuremethylester,
(2-Pyridin-4-yl-imidazo[1,2-a]pyrazin-3-yl)-(1,1,3,3-tetramethyl-butyl)-amin,
(2-Methyl-imidazo[1,2-a]pyrazin-3-yl)-(1,1,3,3-tetramethyl-butyl)-amin,
3-(3-*tert*-Butylamino-imidazo[1,2-a]pyridin-2-yl)-phenol,
Butyl-[2-(2,3-dichlor-phenyl)-imidazo[1,2-a]pyrazin-3-yl]-amin,
[(2-Phenyl-imidazo[1,2-a]pyridin-3-ylamino)-methyl]-phosphonsäurediethylester,
*tert*-Butyl-(2-*tert*-butyl-imidazo[1,2-a]pyridin-3-yl)-amin,
Butyl-(2-o-tolyl-imidazo[1,2-a]pyrimidin-3-yl)-amin,
(2,6-Dimethyl-phenyl)-[2-(2-methoxy-phenyl)-imidazo[1,2-a]pyrazin-3-yl]-amin,
Butyl-(2-o-tolyl-imidazo[1,2-a]pyrimidin-3-yl)-amin,
*tert*-Butyl-(2-pyridin-3-yl-imidazo[1,2-a]pyrimidin-3-yl)-amin,
*tert*-Butyl-(2-methyl-imidazo[1,2-a]pyridin-3-yl)-amin,
[2-(1H-Pyrrol-2-yl)-imidazo[1,2-a]pyrimidin-3-yl]-(1,1,3,3-tetramethyl-butyl)-amin,
Cyclohexyl-(2-furan-2-yl-imidazo[1,2-a]pyridin-3-yl)-amin,
*tert*-Butyl-(2-pyridin-3-yl-imidazo[1,2-a]pyridin-3-yl)-amin,
*tert*-Butyl-(2-pyridin-2-yl-imidazo[1,2-a]pyridin-3-yl)-amin,
*tert*-Butyl-(2-thiophen-2-yl-imidazo[1,2-a]pyridin-3-yl)-amin,
Cyclohexyl-(2-methyl-imidazo[1,2-a]pyridin-3-yl)-amin,
N-Cyclohexyl-N-[2-(5-methyl-furan-2-yl)-imidazo[1,2-a]pyridin-3-yl]-acetamid,
*tert*-Butyl-[2-(5-methylsulfanyl-thiophen-2-yl)-imidazo[1,2-a]pyrimidin-3-yl]-amin,
[2-(3-Brom-thiophen-2-yl)-imidazo[1,2-a]pyridin-3-yl]-cyclohexyl-amin,
Essigsäure-2-methoxy-4-[3-(1,1,3,3-tetramethylbutylamino)-imidazo[1,2-a]pyrimidin-2-yl]-phenylester,
[2-(2-Chlor-4-fluor-phenyl)-imidazo[1,2-a]pyrimidin-3-yl]-(1,1,3,3-tetramethylbutyl)-amin,
(2-Anthracen-9-yl-imidazo[1,2-a]pyrazin-3-yl)-*tert*-butyl-amin,
*tert*-Butyl-(2-naphthalin-1-yl-imidazo[1,2-a]pyridin-3-yl)-amin,
N-Cyclohexyl-N-[2-(4,5-dimethyl-furan-2-yl)-imidazo[1,2-a]pyrimidin-3-yl]-acetamid oder (1,1,3,3-Tetramethylbutyl)-[2-(3,4,5-trimethoxyphenyl)-imidazo[1,2-a]pyridin-3-yl]-amin, handelt.

**4.** Arzneimittel enthaltend als Wirkstoff mindestens ein bicyclisches Imidazo-3-yl-amin der allgemeinen Formel I gemäß Anspruch 1 , in der $R^1$, R2, R3, X und Y die in Anspruch 1 angegebene Bedeutung haben,in Form der Base oder eines pharmazeutisch akzeptablen Salzes.

**5.** Arzneimittel gemäß Anspruch 4, **dadurch gekennzeichnet, daß** es als Wirkstoff mindestens ein bicyclisches Imidazo-3-yl-amin ausgewählt aus der Gruppe

(6-Isocyano-hexyl)-(2-pyridin-2-yl-imidazo(1,2-a]pyridin-3-yl)-amin,
(2-Furan-2-yl-imidazo[1,2-a]pyridin-3-yl)-(6-isocyano-hexyl)-amin,
(2-Cyclohexyl-imidazo[1,2-a]pyrazin-3-yl)-(6-isocyano-hexyl)-amin,
(2,6-Dimethyl-phenyl)-(2-furan-2-yl-imidazo[1,2-a]pyridin-3-yl)-amin,
(2-Furan-2-yl-imidazo[1,2-a]pyrazin-3-ylamino)-essigsäuremethylester,
(2-Cyclohexyl-imidazo[1,2-a]pyrimidin-3-ylamino)-essigsäuremethylester,
(2-Methyl-imidazo[1,2-a]pyrazin-3-ylamino)-essigsäuremethylester,
(2-Pyridin-4-yl-imidazo[1,2-a]pyrazin-3-yl)-(1,1,3,3-tetramethyl-butyl)-amin,
(2-Methyl-imidazo[1,2-a]pyrazin-3-yl)-(1,1,3,3-tetramethyl-butyl)-amin,
3-(3-*tert*-Butylamino-imidazo[1,2-a]pyridin-2-yl)-phenol,
Butyl-[2-(2,3-dichlor-phenyl)-imidazo[1,2-a]pyrazin-3-yl]-amin,
[(2-Phenyl-imidazo[1,2-a]pyridin-3-ylamino)-methyl]-phosphonsäurediethylester,
*tert*-Butyl-(2-*tert*-butyl-imidazo[1,2-a]pyridin-3-yl)-amin,
Butyl-(2-o-tolyl-imidazo[1,2-a]pyrimidin-3-yl)-amin,
(2,6-Dimethyl-phenyl)-[2-(2-methoxy-phenyl)-imidazo[1,2-a]pyrazin-3-yl]-amin,
Butyl-(2-o-tolyl-imidazo[1,2-a]pyrimidin-3-yl)-amin,
*tert*-Butyl-(2-pyridin-3-yl-imidazo[1,2-a]pyrimidin-3-yl)-amin,
*tert*-Butyl-(2-methyl-imidazo[1,2-a]pyridin-3-yl)-amin,
[2-(1H-Pyrrol-2-yl)-imidazo[1,2-a]pyrimidin-3-yl]-(1,1,3,3-tetramethyl-butyl)-amin,
Cyclohexyl-(2-furan-2-yl-imidazo[1,2-a]pyridin-3-yl)-amin,
*tert*-Butyl-(2-pyridin-3-yl-imidazo[1,2-a]pyridin-3-yl)-amin,
*tert*-Butyl-(2-pyridin-2-yl-imidazo[1,2-a]pyridin-3-yl)-amin,
*tert*-Butyl-(2-thiophen-2-yl-imidazo[1,2-a]pyridin-3-yl)-amin,
Cyclohexyl-(2-methyl-imidazo[1,2-a]pyridin-3-yl)-amin,
N-Cyclohexyl-N-[2-(5-methyl-furan-2-yl)-imidazo[1,2-a]pyridin-3-yl]-acetamid,
*tert*-Butyl-[2-(5-methylsulfanyl-thiophen-2-yl)-imidazo[1,2-a]pyrimidin-3-yl]-amin,
[2-(3-Brom-thiophen-2-yl)-imidazo[1,2-a]pyridin-3-yl]-cyclohexyl-amin,
Essigsäure-2-methoxy-4-[3-(1,1,3,3-tetramethylbutylamino)-imidazo[1,2-a]pyrimidin-2-yl]-phenylester,
[2-(2-Chlor-4-fluor-phenyl)-imidazo[1,2-a]pyrimidin-3-yl]-(1,1,3,3-tetramethylbutyl)-amin,
(2-Anthracen-9-yl-imidazo[1,2-a]pyrazin-3-yl)-tert-butyl-amin,
*tert*-Butyl-(2-naphthalin-1-yl-imidazo[1,2-a]pyridin-3-yl)-amin,
N-Cyclohexyl-N-[2-(4,5-dimethyl-furan-2-yl)-imidazo[1,2-a]pyrimidin-3-yl]-acetamid oder (1,1,3,3-Tetramethylbutyl)-[2-(3,4,5-trimethoxyphenyl)-imidazo[1,2-a]pyridin-3-yl]-amin,

oder der pharmazeutisch akzeptablen Salze dieser Verbindungen enthält.

**6.** Verwendung von mindestens einem bicyclischen Imidazo-3-yl-amin gemäß Anspruch 1, 2 oder 3 zusammen mit einem oder mehreren Hilfsstoffen zur Herstellung eines Arzneimittels zur Bekämpfung von Schmerz.

**7.** Verfahren zur Herstellung von bicyclischen Imidazo-3-yl-aminen gemäß Anspruch 1, 2 oder 3 durch Dreikomponentenreaktion aus Amidin, Aldehyd und Isonitril, **dadurch gekennzeichnet, daß** die Synthese der Verbindungen in Dichlormethan als Lösungsmittel und in Gegenwart von Perchlorsäure erfolgt, wobei die Ausgangsverbindungen nacheinander in der Reihenfolge Amidin, Aldehyd und Isonitril zugegeben werden und die entstehenden Produkte gegebenenfalls anschließend mit einer Verbindung $R^2$Hal oder einem Isocyanat $R^e$NCO umgesetzt werden.

## Claims

**1.** Bicyclic imidazo-3-yl-amines of the general formula I

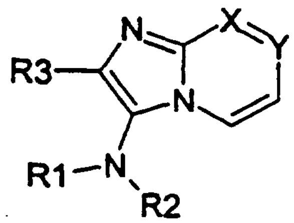

I

wherein

X and Y denote CH or N, with the proviso that X and Y do not simultaneously denote N,

$R^1$ denotes *tert*-butyl, $(CH_2)_nCN$, where n = 4, 5 or 6, optionally substituted phenyl, $C_4$-$C_8$-cycloalkyl, $CH_2CH_2R$ (R = 4-morpholino), 1,1,3,3-tetramethylbutyl or $CH_2R^a$, wherein $R^a$ represents hydrogen, OH, $C_1$-$C_8$-alkyl (branched or unbranched), optionally substituted phenyl, CO(OR') (where R' = unbranched $C_1$-$C_4$-alkyl or branched $C_1$-$C_5$-alkyl), $PO(OR')_2$ (where R' = unbranched $C_1$-$C_4$-alkyl or branched $C_1$-$C_5$-alkyl) or $Si(R^xR^yR^z)$ (where $R^x$, $R^y$ and $R^z$ in each case independently of one another are $C_1$-$C_4$-alkyl (branched or unbranched), $C_4$-$C_8$-cycloalkyl or phenyl),

$R^2$ denotes hydrogen, $COR^b$, wherein $R^b$ represents $C_1$-$C_4$-alkyl (branched or unbranched) or $C_3$-$C_8$-cycloalkyl, $CH_2CH_2CO(OR^c)$, wherein $R^c$ represents $C_1$-$C_4$-alkyl (branched or unbranched), adamantyl, optionally substituted phenyl, optionally substituted 1-naphthyl or 2-naphthyl or in each case optionally substituted 2-pyridyl, 3-pyridyl, 4-pyridyl, thiazolyl or furoyl, $CH_2$phenyl, $CH_2CH_2R^d$, wherein $R^d$ represents optionally substituted phenyl, or $CONHR^e$, wherein $R^e$ represents $C_1$-$C_8$-alkyl (branched or unbranched), $C_3$-$C_8$-cycloalkyl or optionally substituted phenyl,

$R^3$ denotes methyl, ethyl, *tert*-butyl, $C_3$-$C_8$-cycloalkyl, phenyl, optionally monosubstituted in the 3-, 5- or 6-position or optionally polysubstituted in the 4-position and additionally in the 2- and/or 3- and/or 5- and/or 6-position, phenoxy, optionally substituted naphthyl, optionally substituted pyrrole, optionally substituted pyridyl, optionally substituted furan, optionally substituted thiophene, optionally substituted anthracene, optionally substituted phenanthrene or optionally substituted quinoline,

with the proviso that $R^3$ does not denote n-propyl, cyclohexyl, unsubstituted phenyl or phenyl monosubstituted in the 3-position with a carboxylic acid amide group if $R^1$ denotes *tert*-butyl, n-propyl, n-butyl, 1,1,3,3-tetramethylbutyl, cyclohexyl, $CH_2CH_2R$ (R = 4-morpholino), monosubstituted phenyl, 2,6-dimethylphenyl or benzyl and at the same time $R^2$ denotes hydrogen or -CO(methyl), and that $R^2$ does not denote hydrogen if at the same time $R^1$ denotes benzyl and $R^3$ denotes methyl, or at the same time $R^1$ denotes $CH_2C(O)$*tert*-butyl and $R^3$ denotes unsubstituted phenyl, in the form of the bases or of pharmaceutically acceptable salts.

**2.** Bicyclic imidazo-3-yl-amines according to claim 1, **characterized in that** $R^2$ denotes hydrogen, $R^1$ is selected from the group consisting of $(CH_2)_nCN$, where n = 4, 5 or 6, cyclohexyl, $CH_2CO$(Omethyl), 2,6-dimethylphenyl, 1,1,3,3-tetramethylbutyl, *tert*-butyl or n-butyl and $R^3$ is selected from the group consisting of 2-pyridyl, 3-pyridyl, 2-furanyl, 2-pyrroyl, methyl, *tert*-butyl, 3-hydroxyphenyl, 3,4-dimethoxyphenyl, 2,3-dichlorophenyl, 2,4-dichlorophenyl, 2-methoxyphenyl, 2,3-dimethoxyphenyl, 3-bromophenyl, 4-bromo-2-fluorophenyl, 5-bromo-2-fluorophenyl, 3-bromo-4-fluorophenyl, 3-chlorophenyl, 3,4-dichlorophenyl, 3-fluorophenyl, 3-methylphenyl, 3-phenoxyphenyl, 3-(4-chlorophenoxy)phenyl, 2-chloro-4-fluorophenyl, 2-chloro-6-fluorophenyl, 2,4-dimethylphenyl, 2,5-dimethylphenyl, 2-bromophenyl, 2-fluorophenyl or 2-(trifluoromethyl)-phenyl.

**3.** Bicyclic imidazo-3-yl-amines according to claim 1 or 2, **characterized in that** they are

(6-isocyano-hexyl)-(2-pyridin-2-yl-imidazo[1,2-a]pyridin-3-yl)-amine,
(2-furan-2-yl-imidazo[1,2-a]pyridin-3-yl)-(6-isocyano-hexyl)-amine,

(2-cyclohexyl-imidazo[1,2-a]pyrazin-3-yl)-(6-isocyano-hexyl)-amine,
(2,6-dimethyl-phenyl)-(2-furan-2-yl-imidazo[1,2-a]pyridin-3-yl)-amine,
(2-furan-2-yl-imidazo[1,2-a]pyrazin-3-ylamino)-acetic acid methyl ester,
(2-cyclohexyl-imidazo[1,2-a]pyrimidin-3-ylamino)-acetic acid methyl ester,
(2-methyl-imidazo[1,2-a]pyrazin-3-ylamino)-acetic acid methyl ester,
(2-pyridin-4-yl-imidazo[1,2-a]pyrazin-3-yl)-(1,1,3,3-tetramethyl-butyl)-amine,
(2-methyl-imidazo[1,2-a]pyrazin-3-yl)-(1,1,3,3-tetramethyl-butyl)-amine,
3-(3-*tert*-butylamino-imidazo[1,2-a]pyridin-2-yl)-phenol,
butyl-[2-(2,3-dichloro-phenyl)-imidazo[1,2-a]pyrazin-3-yl]-amine,
[(2-phenyl-imidazo[1,2-a]pyridin-3-ylamino)-methyl]-phosphonic acid diethyl ester,
*tert*-butyl-(2-*tert*-butyl-imidazo[1,2-a]pyridin-3-yl)-amine,
butyl-(2-o-tolyl-imidazo[1,2-a]pyrimidin-3-yl)-amine, (2,6-dimethyl-phenyl)-[2-(2-methoxy-phenyl)-imidazo [1,2-a]pyrazin-3-yl]-amine,
butyl-(2-o-tolyl-imidazo[1,2-a]pyrimidin-3-yl)-amine, *tert*-butyl-(2-pyridin-3-yl-imidazo[1,2-a]pyrimidin-3-yl)-amine,
*tert*-butyl-(2-methyl-imidazo[1,2-a]pyridin-3-yl)-amine,
[2-(1H-pyrrol-2-yl)-imidazo[1,2-a]pyrimidin-3-yl]-(1,1,3,3-tetramethyl-butyl)-amine,
cyclohexyl-(2-furan-2-yl-imidazo[1,2-a]pyridin-3-yl)-amine,
*tert*-butyl-(2-pyridin-3-yl-imidazo[1,2-a]pyridin-3-yl)-amine,
*tert*-butyl-(2-pyridin-2-yl-imidazo[1,2-a]pyridin-3-yl)-amine,
*tert*-butyl-(2-thiophen-2-yl-imidazo[1,2-a]pyridin-3-yl)-amine,
cyclohexyl-(2-methyl-imidazo[1,2-a]pyridin-3-yl)-amine,
N-cyclohexyl-N-[2-(5-methyl-furan-2-yl)-imidazo[1,2-a]pyridin-3-yl]-acetamide,
*tert*-butyl-[2-(5-methylsulfanyl-thiophen-2-yl)-imidazo[1,2-a]pyrimidin-3-yl]-amine,
[2-(3-bromo-thiophen-2-yl)-imidazo[1,2-a]pyridin-3-yl]-cyclohexyl-amine,
acetic acid 2-methoxy-4-[3-(1,1,3,3-tetramethylbutylamino)-imidazo[1,2-a]pyrimidin-2-yl]-phenyl ester,
[2-(2-chloro-4-fluoro-phenyl)-imidazo[1,2-a]pyrimidin-3-yl]-(1,1,3,3-tetramethylbutyl)-amine,
(2-anthracen-9-yl-imidazo[1,2-a]pyrazin-3-yl)-*tert*-butyl-amine,
*tert*-butyl-(2-naphthalen-1-yl-imidazo[1,2-a]pyridin-3-yl)-amine,
N-cyclohexyl-N-[2-(4,5-dimethyl-furan-2-yl)-imidazo[1,2-a]pyrimidin-3-yl]-acetamide or (1,1,3,3-tetramethyl-butyl)-[2-(3,4,5-trimethoxyphenyl)-imidazo[1,2-a]pyridin-3-yl]-amine.

**4.** Medicaments comprising as the active compound at least one bicyclic imidazo-3-yl-amine of the general formula I according to claim 1, in which $R^1$, $R^2$, $R^3$, X and Y have the meaning given in claim 1, in the form of the base or of a pharmaceutically acceptable salt.

**5.** Medicament according to claim 4, **characterized in that** it comprises as the active compound at least one bicyclic imidazo-3-yl-amine selected from the group consisting of

(6-isocyano-hexyl)-(2-pyridin-2-yl-imidazo[1,2-a]pyridin-3-yl)-amine,
(2-furan-2-yl-imidazo[1,2-a]pyridin-3-yl)-(6-isocyano-hexyl)-amine,
(2-cyclohexyl-imidazo[1,2-a]pyrazin-3-yl)-(6-isocyano-hexyl)-amine,
(2,6-dimethyl-phenyl)-(2-furan-2-yl-imidazo[1,2-a]pyridin-3-yl)-amine,
(2-furan-2-yl-imidazo[1,2-a]pyrazin-3-ylamino)-acetic acid methyl ester,
(2-cyclohexyl-imidazo[1,2-a]pyrimidin-3-ylamino)-acetic acid methyl ester,
(2-methyl-imidazo[1,2-a]pyrazin-3-ylamino)-acetic acid methyl ester,
(2-pyridin-4-yl-imidazo[1,2-a]pyrazin-3-yl)-(1,1,3,3-tetramethyl-butyl)-amine,
(2-methyl-imidazo[1,2-a]pyrazin-3-yl)-(1,1,3,3-tetramethyl-butyl)-amine,
3-(3-tert-butylamino-imidazo[1,2-a]pyridin-2-yl)-phenol,
butyl-[2-(2,3-dichloro-phenyl)-imidazo[1,2-a]pyrazin-3-yl]-amine,
[(2-phenyl-imidazo[2,2-a]pyridin-3-ylamino)-methyl]-phosphonic acid diethyl ester,
*tert*-butyl-(2-*tert*-butyl-imidazo[1,2-a]pyridin-3-yl)-amine,
butyl-(2-o-tolyl-imidazo[1,2-a]pyrimidin-3-yl)-amine, (2,6-dimethyl-phenyl)-[2-(2-methoxy-phenyl)-imidazo [1,2-a]pyrazin-3-yl]-amine,
butyl-(2-o-tolyl-imidazo[1,2-a]pyrimidin-3-yl)-amine, *tert*-butyl-(2-pyridin-3-yl-imidazo[1,2-a]pyrimidin-3-yl)-amine,
*tert*-butyl-(2-methyl-imidazo[1,2-a]pyridin-3-yl)-amine,
[2-(1H-pyrrol-2-yl)-imidazo[1,2-a]pyrimidin-3-yl]-(1,1,3,3-tetramethyl-butyl)-amine,

cyclohexyl-(2-furan-2-yl-imidazo[1,2-a]pyridin-3-yl)-amine,
*tert*-butyl-(2-pyridin-3-yl-imidazo[1,2-a]pyridin-3-yl)-amine,
*tert*-butyl-(2-pyridin-2-yl-imidazo[1,2-a]pyridin-3-yl)-amine,
*tert*-butyl-(2-thiophen-2-yl-imidazo[1,2-a]pyridin-3-yl)-amine,
cyclohexyl-(2-methyl-imidazo[1,2-a]pyridin-3-yl)-amine,
N-cyclohexyl-N-[2-(5-methyl-furan-2-yl)-imidazo[1,2-a]pyridin-3-yl]-acetamide,
*tert*-butyl-[2-(5-methylsulfanyl-thiophen-2-yl)-imidazo[1,2-a]pyrimidin-3-yl]-amine,
[2-(3-bromo-thiophen-2-yl)-imidazo[1,2-a]pyridin-3-yl]-cyclohexyl-amine,
acetic acid 2-methoxy-4-[3-(1,1,3,3-tetramethylbutylamino)-imidazo[1,2-a]pyrimidin-2-yl]-phenyl ester,
[2-(2-chloro-4-fluoro-phenyl)-imidazo[1,2-a]pyrimidin-3-yl]-(1,1,3,3-tetramethylbutyl)-amine,
(2-anthracen-9-yl-imidazo[1,2-a]pyrazin-3-yl)-*tert*-butyl-amine,
*tert*-butyl-(2-naphthalen-1-yl-imidazo[1,2-a]pyridin-3-yl)-amine,
N-cyclohexyl-N-(2-(4,5-dimethyl-furan-2-yl)-imidazo[1,2-a]pyrimidin-3-yl]-acetamide or (1,1,3,3-tetramethylbutyl)-[2-(3,4,5-trimethoxyphenyl)-imidazo[1,2-a]pyridin-3-yl]-amine

or the pharmaceutically acceptable salts of these compounds.

**6.** Use of at least one bicyclic imidazo-3-yl-amine according to claim 1, 2 or 3 together with one or more auxiliary substances for the preparation of a medicament for combating pain.

**7.** Process for the preparation of bicyclic imidazo-3-yl-amines according to claim 1, 2 or 3 by a three-component reaction from amidine, aldehyde and isonitrile, **characterized in that** the synthesis of the compounds is carried out in methylene chloride as the solvent and in the presence of perchloric acid, the starting compounds being added in succession in the sequence amidine, aldehyde and isonitrile and the products formed optionally then being reacted with a compound $R^2Hal$ or an isocyanate $R^eNCO$.

## Revendications

**1.** Imidazo-3-yl-amines bicycliques de formule générale I,

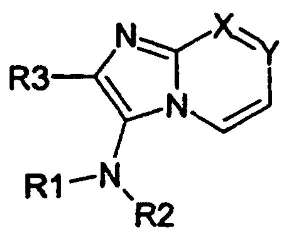

I

dans laquelle

X et Y représentent CH ou N, sous réserve que X et Y ne représentent pas N en même temps,
$R^1$ est un reste tertiobutyle, $(CH_2)_nCN$ où n a la valeur 4, 5 ou 6, un reste phényle éventuellement substitué, cycloalkyle en $C_4$ à $C_8$, $CH_2CH_2R$ (R = 4-morpholino), 1,1,3,3-tétraméthylbutyle ou $CH_2R^a$, où $R^a$ est l'hydrogène, un radical OH, alkyle en $C_1$ à $C_8$ (ramifié ou non ramifié), phényle éventuellement substitué, CO(OR') (où R' = alkyle en $C_1$ à $C_4$ non ramifié ou alkyle en $C_1$ à $C_5$ ramifié), $PO(OR')_2$ (où R' = alkyle en $C_1$ à $C_4$ non ramifié ou alkyle en $C_1$ à $C_5$ ramifié) ou $Si(R^xR^yR^z)$ (où $R^x$, $R^y$ et $R^z$ représentent chacun indépendamment l'un de l'autre un radical alkyle en $C_1$ à $C_4$ (ramifié ou non ramifié), cycloalkyle en $C_4$ à $C_8$ ou phényle),
$R^2$ est l'hydrogène, un reste $COR^b$, où $R^b$ est un radical alkyle en $C_1$ à $C_4$ (ramifié ou non ramifié) ou cycloalkyle en $C_3$ à $C_8$, un reste $CH_2CH_2CO(OR^c)$, où $R^c$ est un radical alkyle en $C_1$ à $C_4$ (ramifié ou non ramifié), adamantyle, phényle éventuellement substitué, 1-naphtyle éventuellement substitué ou 2-naphtyle ou un radical 2-pyridyle, 3-pyridyle, 4-pyridyle, thiazolyle ou furoyle dont chacun est éventuellement substitué, un reste $CH_2$phényle, $CH_2CH_2R^d$, où $R^d$ est un radical phényle éventuellement substitué, ou un reste $CONHR^e$, où $R^e$

est un radical alkyle en $C_1$ à $C_8$ (ramifié ou non ramifié), cycloalkyle en $C_3$ à $C_8$ ou phényle éventuellement substitué,
$R^3$ est un reste méthyle, éthyle, tertiobutyle, cycloalkyle en $C_3$ à $C_8$, phényle, éventuellement monosubstitué en position 3, 5 ou 6 ou éventuellement polysubstitué en position 4 et en outre en positions 2 et/ou 3 et/ou 5 et/ou 6, un reste phénoxy, naphtyle éventuellement substitué, pyrrole éventuellement substitué, pyridyle éventuellement substitué, furanne éventuellement substitué, thiophène éventuellement substitué, anthracène éventuellement substitué, phénanthrène éventuellement substitué ou quinoléine éventuellement substitué,

sous réserve que $R^3$ ne soit pas un reste n-propyle, cyclohexyle, phényle non substitué ou phényle monosubstitué en position 3 avec un groupe amide d'acide carboxylique lorsque $R_1$ est un reste tertiobutyle, n-propyle, n-butyle,
1,1,3,3-tétraméthylbutyle, cyclohexyle, $CH_2CH_2R$ (R = 4-morpholino), phényle monosubstitué, 2,6-diméthylphényle ou benzyle en même temps que $R^2$ est l'hydrogène ou un reste -CO(méthyle), et sous réserve que $R^2$ ne soit pas l'hydrogène lorsque, en même temps, $R^1$ est un reste benzyle et $R^3$ est un reste méthyle ou bien, en même temps, $R^1$ est un reste $CH_2C(O)$tertiobutyle et $R^3$ est un reste phényle non substitué,
sous forme des bases ou de sels acceptables du point de vue pharmaceutique.

2. Imidazo-3-yl-amines bicycliques suivant la revendication 1, **caractérisées en ce que** $R^2$ désigne l'hydrogène,
$R^1$ est choisi dans le groupe des restes $(CH_2)_nCN$ où n a la valeur 4, 5 ou 6, cyclohexyle, $CH_2CO$(Ométhyle), 2,6-diméthylphényle, 1,1,3,3-tétraméthylbutyle, tertiobutyle ou n-butyle et
$R^3$ est choisi dans le groupe des restes 2-pyridyle, 3-pyridyle, 2-furannyle, 2-pyrroyle, méthyle, tertiobutyle, 3-hydroxyphényle, 3,4-diméthoxyphényle, 2,3-dichlorophényle, 2,4-dichlorophényle, 2-méthoxyphényle, 2,3-diméthoxyphényle, 3-bromophényle, 4-bromo-2-fluorophényle, 5-bromo-2-fluorophényle, 3-bromo-4-fluorophényle, 3-chlorophényle, 3,4-dichlorophényle, 3-fluorophényle, 3-méthylphényle, 3-phénoxyphényle, 3-(4-chlorophénoxy) phényle, 2-chloro-4-fluorophényle, 2-chloro-6-fluorophényle, 2,4-diméthylphényle, 2,5-diméthylphényle, 2-bromophényle, 2-fluorophényle ou 2-(trifluorométhyl)-phényle.

3. Imidazo-3-yl-amines bicycliques suivant la revendication 1 ou 2, **caractérisées en ce qu'**il s'agit des composés suivants :

(6-isocyano-hexyl)-(2-pyridine-2-yl-imidazo[1,2-a]pyridine-3-yl)-amine,
(2-furanne-2-yl-imidazo[1,2-a]pyridine-3-yl)-(6-isocyano-hexyl)-amine,
(2-cyclohexyl-imidazo[1,2-a]pyrazine-3-yl)-(6-isocyano-hexyl)-amine,
(2,6-diméthylphényl)-(2-furanne-2-yl-imidazo[1,2-a]-pyridine-3-yl)-amine,
(2-furanne-2-yl-imidazo[1,2-a]pyrazine-3-ylamino)-acétate de méthyle,
(2-cyclohexyl-imidazo[1,2-a]pyrimidine-3-ylamino)-acétate de méthyle,
(2-méthyl-imidazo[1,2-a]pyrazine-3-ylamino)-acétate de méthyle,
(2-pyridine-4-yl-imidazo[1,2-a]pyrazine-3-yl)-(1,1,3,3-tétraméthylbutyl)-amine,
(2-méthyl-imidazo[1,2-a]pyrazine-3-yl)-(1,1,3,3-tétra-méthylbutyl)-amine,
3-(3-tertiobutylamino-imidazo[1,2-a]pyridine-2-yl)-phénol,
butyl-[2-(2,3-dichlorophényl)-imidazo[1,2-a]pyrazine-3-yl]-amine,
[(2-phénylimidazo[1,2-a]pyridine-3-ylamino)-méthyl]-phosphonate de diéthyle,
tertiobutyl-(2-tertiobutylimidazo[1,2-a]pyridine-3-yl)-amine,
butyl-(2-o-tolyl-imidazo[1,2-a]pyrimidine-3-yl)-amine, (2,6-diméthylphényl)-[2-(2-méthoxyphényl)-imidazo[1,2-a]pyrazine-3-yl]-amine,
butyl-(2-o-tolyl-imidazo[1,2-a]pyrimidine-3-yl)-amine, tertiobutyl-(2-pyridine-3-yl-imidazo[1,2-a]pyrimidine-3-yl)-amine,
tertiobutyl-(2-méthylimidazo[1,2-a]pyridine-3-yl)-amine, [2-(1H-pyrrole-2-yl)-imidazo[1,2-a]pyrimidine-3-yl]-(1,1,3,3-tétraméthylbutyl)-amine,
cyclohexyl-(2-furanne-2-yl-imidazo[1,2-a]pyridine-3-yl)-amine,
tertiobutyl-(2-pyridine-3-yl-imidazo[1,2-a]pyridine-3-yl)-amine,
tertiobutyl-(2-pyridine-2-yl-imidazo[1,2-a]pyridine-3-yl)-amine,
tertiobutyl-(2-thiophène-2-yl-imidazo[1,2-a]pyridine-3-yl)-amine,
cyclohexyl-(2-méthyl-imidazo[1,2-a]pyridine-3-yl)-amine,
N-cyclohexyl-N-[2-(5-méthyl-furanne-2-yl)-imidazo[2,2-a]-pyridine-3-yl]-acétamide,
tertiobutyl-[2-(5-méthylsulfanyl-thiophène-2-yl)-imidazo-[1,2-a]pyrimidine-3-yl]-amine,
[2-(3-bromo-thiophène-2-yl)-imidazo[1,2-a]pyridine-3-yl]-cyclohexylamine,
2-méthoxy-4-[3-(1,1,3,3-tétraméthylbutylamino)-imidazo-[1,2-a]pyrimidine-2-yl]-acétate de phényle,

[2-(2-chloro-4-fluorophényl)-imidazo[1,2-a]pyrimidine-3-yl]-(1,1,3,3-tétraméthylbutyl)-amine,
(2-anthracène-9-yl-imidazo[1,2-a]pyrazine-3-yl)-tertio-butylamine,
tertiobutyl-(2-naphtalène-1-yl-imidazo[1,2-a]pyridine-3-yl)-amine,
N-cyclohexyl-N-[2-(4,5-diméthyl-furanne-2-yl)-imidazo[1,2-a]pyrimidine-3-yl]-acétamide ou
(1,1,3,3-tétraméthylbutyl)-[2-(3,4,5-triméthoxyphényl)-imidazo[1,2-a]pyridine-3-yl]-amine.

**4.** Médicament contenant comme substance active au moins une imidazo-3-yl-amine bicyclique de formule générale I suivant la revendication 1, dans laquelle $R^1$, $R^2$, $R^3$, X et Y ont la définition indiquée dans la revendication 1, sous forme de la base ou d'un sel acceptable du point de vue pharmaceutique.

**5.** Médicament suivant la revendication 4, **caractérisé en ce qu'**il contient comme substance active au moins une imidazo-3-yl-amine bicyclique choisie dans le groupe des composés suivants :

(6-isocyano-hexyl)-(2-pyridine-2-yl-imidazo[1,2-a]pyridine-3-yl)-amine,
(2-furanne-2-yl-imidazo[1,2-a]pyridine-3-yl)-(6-isocyano-hexyl)-amine,
(2-cyclohexyl-imidazo[1,2-a]pyrazine-3-yl)-(6-isocyano-hexyl)-amine,
(2,6-diméthylphényl)-(2-furanne-2-yl-imidazo[1,2-a]-pyridine-3-yl)-amine,
(2-furanne-2-yl-imidazo[1,2-a]pyrazine-3-ylamino)-acétate de méthyle,
(2-cyclohexyl-imidazo[1,2-a]pyrimidine-3-ylamino)-acétate de méthyle,
(2-méthyl-imidazo[1,2-a]pyrazine-3-ylamino)-acétate de méthyle,
(2-pyridine-4-yl-imidazo[1,2-a]pyrazine-3-yl)-(1,1,3,3-tétraméthylbutyl)-amine,
(2-méthyl-imidazo[1,2-a]pyrazine-3-yl)-(1,1,3,3-tétraméthylbutyl)-amine,
3-(3-tertiobutylamino-imidazo[1,2-a]pyridine-2-yl)-phénol,
butyl-[2-(2,3-dichlorophényl)-imidazo[1,2-a]pyrazine-3-yl]-amine,
[(2-phénylimidazo[1,2-a]pyridine-3-ylamino)-méthyl]-phosphonate de diéthyle,
tertiobutyl-(2-tertiobutylimidazo[1,2-a]pyridine-3-yl)-amine,
butyl-(2-o-tolyl-imidazo[1,2-a]pyrimidine-3-yl)-amine,
(2,6-diméthylphényl)-[2-(2-méthoxyphényl)-imidazo[1,2-a]-pyrazine-3-yl]-amine,
butyl-(2-o-tolyl-imidazo[1,2-a]pyrimidine-3-yl)-amine, tertiobutyl-(2-pyridine-3-yl-imidazo[1,2-a]pyrimidine-3-yl)-amine,
tertiobutyl-(2-méthylimidazo[1,2-a]pyridine-3-yl)-amine, [2-(1H-pyrrole-2-yl)-imidazo[1,2-a]pyrimidine-3-yl]-(1,1,3,3-tétraméthylbutyl)-amine,
cyclohexyl-(2-furanne-2-yl-imidazo[1,2-a]pyridine-3-yl)-amine,
tertiobutyl-(2-pyridine-3-yl-imidazo[1,2-a]pyridine-3-yl)-amine,
tertiobutyl-(2-pyridine-2-yl-imidazo[1,2-a]pyridine-3-yl)-amine,
tertiobutyl-(2-thiophène-2-yl-imidazo[2,2-a]pyridine-3-yl)-amine,
cyclohexyl-(2-méthyl-imidazo[1,2-a]pyridine-3-yl)-amine,
N-cyclohexyl-N-[2-(5-méthyl-furanne-2-yl)-imidazo[1,2-a]-pyridine-3-yl]-acétamide,
tertiobutyl-[2-(5-méthylsulfanyl-thiophène-2-yl)-imidazo-[1,2-a]pyrimidine-3-yl]-amine,
[2-(3-bromo-thiophène-2-yl)-imidazo[1,2-a]pyridine-3-yl]-cyclohexylamine,
2-méthoxy-4-[3-(1,1,3,3-tétraméthylbutylamino)-imidazo-[1,2-a]pyrimidine-2-yl]-acétate de phényle,
[2-(2-chloro-4-fluorophényl)-imidazo[1,2-a]pyrimidine-3-yl]-(1,1,3,3-tétraméthylbutyl)-amine,
(2-anthracène-9-yl-imidazo[1,2-a]pyrazine-3-yl)-tertio-butylamine,
tertiobutyl-(2-naphtalène-1-yl-imidazo[1,2-a]pyridine-3-yl)-amine,
N-cyclohexyl-N-[2-(4,5-diméthyl-furanne-2-yl)-imidazo-[1,2-a]pyrimidine-3-yl]-acétamide ou (1,1,3,3-tétraméthyl-butyl)-[2-(3,4,5-triméthoxyphényl)-imidazo[1,2-a]pyridine-3-yl]-amine, ou des sels acceptables du point de vue pharmaceutique de ces composés.

**6.** Utilisation d'au moins une imidazo-3-yl-amine bicyclique suivant la revendication 1, 2 ou 3 conjointement avec une ou plusieurs substances auxiliaires pour la préparation d'un médicament destiné à combattre la douleur.

**7.** Procédé de préparation d'imidazo-3-yl-amines bicycliques suivant la revendication 1, 2 ou 3, par réaction à trois composants à partir d'amidine, d'aldéhyde et d'isonitrile, **caractérisé en ce que** l'on conduit la synthèse des composés dans du dichlorométhane utilisé comme solvant et en présence d'acide perchlorique, en ajoutant les composés de départ successivement dans l'ordre amidine, aldéhyde et isonitrile et, le cas échéant, en faisant réagir ensuite les produits obtenus avec un composé $R^2$Hal ou un isocyanate $R^e$NCO.